# EUROPEAN PATENT SPECIFICATION

(11) **EP 1 273 312 B1**
(45) Date of publication and mention of the grant of the patent: **06.09.2006**
(21) Application number: 02254659.2
(22) Date of filing: 03.07.2002
(51) Int. Cl.: A61L 27/24, A61L 27/38, A61L 27/56, A61L 27/18, A61L 27/44, A61L 27/58

(54) **Implant for cartilage tissue regeneration**
Implantat zur Regeneration von Knorpelgewebe
Implant pour régénération de tissus cartilagineux

(30) Priority: 04.07.2001 JP 2001204013
(43) Date of publication of application: 08.01.2003
(73) Proprietor: National Institute of Advanced Industrial Science and Technology, Tokyo 100-8921 (JP)
(72) Inventor: Chen, Guoping, c/o AIST, Tsukuba-shi, Ibaraki 305-8562 (JP); Ushida, Takashi, Tsukuba-shi, Ibaraki 305-0821 (JP); Tateishi, Tetsuya, Tsukuba-shi, Ibaraki 305-0042 (JP)
(74) Representative: Woods, Geoffrey Corlett

(56) References cited:
- WO-A-01/54735
- WO-A-93/21857
- WO-A-98/40111
- WO-A-99/47186
- US-A- 5 683 459
- US-A- 5 916 585
- US-A- 6 080 194
- FREED L E ET AL: "NEOCARTILAGE FORMATION IN VITRO AND IN VIVO USING CELLS CULTURED ON SYNTHETIC BIODEGRADABLE POLYMERS" JOURNAL OF BIOMEDICAL MATERIALS RESEARCH, WILEY, NEW YORK, NY, US, vol. 27, no. 1, 1993, pages 11-23, XP002072408 ISSN: 0021-9304

## Description

### BACKGROUND OF THE INVENTION

### 1. FIELD OF THE INVENTION

The present invention relates generally to tissue regeneration, particularly cartilage tissue regeneration for repairing cartilage lesion caused by, for example, accidents or diseases, including osteoarthritis.

### 2. DESCRIPTION OF THE RELATED ART

Articular cartilage defect caused by osteoarthritis or traumatic lesions is a major problem in orthopedic surgery due to the limited capacity for repair and self-regeneration. Therapies for cartilage defects include transplantation of autografts, allografts and artificial prosthetic substitutes. Each of these techniques has its specific problems and limitations. Autografts are limited by a lack of an adequate supply of donors and by donor site morbidity. Allografts include problems with the potential transfer of pathogens and tissue rejection. Prosthetic implants have the problems of abrasion, loosing and unknown long-term side effects (Langer, R. et al., Science 1993, 14;260(5110):920-926). Tissue engineering combining biodegradable porous scaffold and chondrocytes or multipotential chondral progenitor cells has emerged as one promising alternative approach for cartilage repair (Boyan, B.D. et al., Clin. Plast. Surg. 1999, 26(4):629-645).

A temporary three-dimensional scaffold is needed to serve as an adhesive substrate to accommodate sufficient cells and to serve as a physical support to guide the formation of the new organs. The scaffold should allow the implanted cells to continue proliferation, secrete extracellular matrices, differentiate, and organize into a new tissue of defined shape. During this process, the scaffold should gradually degrades and is eventually eliminated. Therefore, in addition to facilitating cell adhesion, promoting cell growth, and allowing the retention of differentiated cell functions, the scaffold should be biocompatible, biodegradable, highly porous, mechanically strong, and malleable into desired shapes. Conventionally, the three-dimensional scaffolds can be prepared from synthetic polymer, such as poly(lactic acid) (PLA), poly(glycolic acid) (PGA) and their copolymer of poly(DL-lactic-co-glycolic acid) (PLGA) , and naturally derived polymer such as collagen (Freed, L.E. et al., J. Biomed. Mater. Res. 1993, 27(1):11-23).

Synthetic polymers can be processed into desired shapes with relatively good mechanical strength and their degradation can be manipulated to match the speed of the new tissue formation. However, synthetic polymer-derived scaffolds lack cell-recognition signals and the surfaces of scaffolds are hydrophobic, which hinder smooth cell seeding. And the big openings in the sponge or mesh of synthetic polymer are not benefit to cell loading. Most cells will pass through the big openings. The uniform distribution of sufficient cells throughout the three-dimensional porous scaffold is difficult to be achieved and thus reduce the efficiency of the formation and functions of new tissues. On the other hand, porous three-dimensional scaffolds of naturally derived polymers, for example, collagen sponge, have the advantage of good cell interaction and hydrophilicity, which benefit cell seeding. However, they are mechanically too weak to maintain the desired shape and structures, and so soft as to be easy to twist; thus, they are difficult to handle in clinical applications (Kim, B.S. et al., Trends Biotechnol. 1998, 16 (5) :224-230).

### SUMMARY OF THE INVENTION

The present invention aims to solve the difficulties with prior arts described above.

More specifically, an object of the present invention is to provide a supporting scaffold for easy and even cell seeding of chondrocytes or their progenitor cells to benefit the regeneration of functional cartilage tissue. Further, the scaffold has a high mechanical strength and easy to clinically handle. Another object of the present invention is to provide an implant for cartilage tissue repairing which comprises the porous scaffold and chondrocytes or their progenitor cells differentiating thereto.

Accordingly, the present invention provides a composite material comprising a mesh or porous sponge, of a biodegradable synthetic polymer and a porous sponge of a naturally derived polymer formed on and/or in the mesh or porous sponge, for example in the openings such as the pores of a sponge or interstices of mesh, wherein the composite material is cross-linked. In a preferred embodiment, the composite material according to the invention is in the form of a sheet. In another preferred embodiment, the composite material according to the invention is in the form of a laminate or roll. In a preferred embodiment, the composite material according to the invention comprises a poly(D,L-lactic-co-glycolic acid) mesh and a collagen sponge, which is cross-linked.

The biodegradable synthetic polymer mesh or porous sponge serves as a mechanical skeleton to facilitate formation of the composite material into designed shapes with good mechanical strength, and easy handling. The naturally derived polymer porous sponge contributes good cell interaction and hydrophilicity, and thus easy homogeneous cell seeding of chondrocytes and their progenitor cells. So, the composite material according to the present invention combines the advantages of both synthetic polymers and naturally derived polymers. Further, the composite material in the form of a sheet, in particular, is good in cell seeding efficiency so that sufficient cells can be accommodated homogeneously throughout the porous scaffold. Due to these characteristics, when the chondrocytes or their progenitor cells differentiating thereto are seeded onto the composite material and implanted into the injured cartilage, a new functional cartilage tissue may be promptly regenerated.

Accordingly, the present invention also provides a scaffold for supporting a cell, comprising the composite material according to the present invention. In a preferred embodiment, the scaffold supports chondrocytes or their progenitor cells differentiating thereto.

Further, the present invention provides an implant for use in cartilage tissue regeneration, comprising chondrocytes or their progenitor cells differentiating thereto and a supporting scaffold which comprises the composite material according to the invention.

Therefore, the scaffold and the implant comprising chondrocytes or their progenitor cells differentiating thereto are highly beneficial as means for cartilage tissue regeneration.

Still further, the present invention provides a method for preparing the composite material, comprising:
(a) depositing and impregnating an aqueous solution of a naturally-derived polymer in a mesh or porous sponge of a biodegradable synthetic polymer;
(b) freeze-drying the aqueous solution-impregnated mesh or porous sponge; and
(c) treating the resulting composite material with a gaseous chemical crosslinking agent.

### BRIEF DESCRIPTION OF THE DRAWINGS

Fig. 1 (a) shows a schematic cross section of an example of the sheet form composite materials according to the present invention.
Fig. 1 (b) shows a schematic cross section of another example of the sheet form composite materials according to the present invention.
Fig. 2 shows a schematic cross section of the laminate form composite material according to the present invention.
Fig. 3 shows a schematic cross section of the roll form composite material according to the present invention.
Fig. 4 is an electron micrograph of a sheet form composite material according to the invention.
Fig. 5 is a photograph showing an appearance of a regenerated bovine articular cartilage tissue.
Fig. 6 is a photograph showing histological staining of a regenerated bovine articular cartilage tissue.

### DETAILED DESCRIPTION OF THE INVENTION

The porous scaffold for supporting chondrocytes or their progenitor cells differentiating thereto according to the present invention comprises a composite material of the present invention, which in turn comprises a mesh or porous sponge of a biodegradable synthetic polymer and a porous sponge of a naturally derived polymer formed on and/or in the mesh or porous sponge, wherein the composite material is cross-linked.

The mesh or porous sponge of a biodegradable synthetic polymer (hereinafter also referred to as "biodegradable synthetic polymer mesh or porous sponge") is herein used mainly to increase the mechanical strength of the composite material according to the present invention. The mesh may consist of textile, woven cloth, nonwoven fabric and the like. Such a mesh is well-known in the art and commercially available from a number of suppliers, such as Vicryl knitted mesh from Ethico, INC. (Somerville, New Jersey) . The porous sponge can be prepared by any well-known method including gas foaming molding employing a foaming agent or poregen-leaching method. In the gas foaming molding of porous sponge, a foaming agent such as high pressure gas and gas-generating solid is added to synthetic polymer and expanded after saturation to form the porous sponge. In the latter, a porogen material such as water-soluble sugar or salt is mixed with a solution of synthetic polymer in an organic solvent such as chloroform, the mixture of the synthetic polymer and porogen is cast in a mold with a designed shape and, after drying the mixture, the porogen is leached out by washing with water to generate the pores to form a porous sponge.

The greater the mesh or pore size of the mesh or porous sponge, the higher the pore density in the porous sponge of a naturally derived polymer per unit or pore of the mesh or porous sponge, although the mechanical strength of the composite material decreases. since the cells are seeded and held in the pores within the porous sponge of the naturally derived polymer according to the invention, the number of the cells to be seeded in the composite material can be increased. As a result, cartilage tissues can be more effectively regenerated.

Thus, the mesh or pore size of the mesh or porous sponge can appropriately be determined depending on sites in an organism to be implanted, desired mechanical strength or elasticity, or regeneration rate of cartilage tissues and so forth.

The biodegradable synthetic polymer which forms the mesh or porous sponge may include, but is not limited to, polyesters such as polylactic acid, polyglycolic acid, poly(D,L-lactic-co-glycolic acid) (PLGA), polymalic acid, and poly-ε-caprolactone. Preferably, the biodegradable synthetic polymer used in the invention is polylactic acid, polyglycolic acid, and poly(D,L-lactic- co-glycolic acid).

For the porous sponge of the naturally derived polymer (hereinafter also referred to as "naturally derived polymer porous sponge"), any naturally derived polymer may be employed herein which is recombinantly obtained using an genetic engineering or derived from a living organism and exhibits biocompatibility. Preferably, the naturally derived polymer may be selected from the group consisting of collagen, gelatin, fibronectin, and laminin, with collagen being especially preferred. Collagen includes types I, II, III, and IV, any of which may be used in the present invention. The naturally derived polymer can be used individually or in any combination, for example, mixture of collagen and laminin, or collagen, laminin and fibronectin.

The pores of the naturally derived polymer porous sponge serve as a platform or anchor for the attachment and proliferation of seeded cells and tissue regeneration. The pores may preferably be continuous. The pore size may be 1 - 300 µm, preferably 20 - 100 µm.

In addition, the thickness of the composite material of the invention may appropriately be determined depending on the applications of the composite material of the invention. Generally, the thickness is 0.1 - 5 mm, preferably is 0.1 - 1 mm. Its porosity may be generally 80% or more, and preferably 80-99%.

In the composite material according to the present invention, a porous sponge of the naturally derived polymer is formed on and/or in the mesh or porous sponge, more specifically, in the openings of the porous scaffold of synthetic polymer, for example, the interstices of a mesh or pores of a porous sponge of synthetic polymer. The surfaces of the openings of porous scaffolds of synthetic polymer , for example, the wall surfaces of interstices of a mesh or the wall surfaces of pores of a porous sponge of synthetic polymer, are also coated with naturally-derived polymer. The composite material according to this invention can be produced by a variety of procedures. For example, it can be obtained by introducing the naturally derived polymer porous sponge into the biodegradable synthetic polymer mesh or porous sponge.

The above method comprises the steps of:
(a) depositing and impregnating an aqueous solution of a naturally derived polymer such as collagen in the mesh or porous sponge of biodegradable synthetic polymer;
(b) freeze-drying the aqueous solution-impregnated mesh or porous sponge; and
(c) treating the resulting composite material with a gaseous chemical crosslinking agent.

In the above step (a), the biodegradable synthetic polymer mesh or porous sponge is treated with an aqueous solution comprising the naturally derived polymer. There are various treatment procedures, but preferably dipping or coating is often used.

Dipping is effective when the concentration or viscosity of the aqueous solution comprising the naturally derived polymer is low. More specifically, the biodegradable synthetic polymer mesh or porous sponge is immersed into an aqueous solution of the naturally derived polymer at a low concentration.

Coating is effective when the concentration or viscosity of the aqueous solution comprising the naturally derived polymer is high and dipping can not be applicable. More specifically, the biodegradable synthetic polymer mesh or porous sponge is coated with a high concentration aqueous naturally derived polymer solution.

The resulting composite in which the aqueous naturally derived polymer solution is impregnated in or deposited on the biodegradable synthetic polymer mesh or porous sponge is then subjected to the freeze-drying step (b).

Freeze-drying step is to freeze the above composite and freeze-dry it in vacuo, and this step allows the naturally derived polymer to become porous, forming a composite material comprising the biodegradable synthetic polymer mesh or porous sponge and the naturally derived polymer porous sponge.

As a procedure for freeze-drying, any conventionally well-known method can be applicable as it is. The temperature during freeze-drying is usually set below -20 °C. The freeze-dry pressure may be set to a reduced pressure condition such that frozen water can vaporize into a gas, and is usually adjusted to reduced pressures of around 0.2 Torr.

The freeze-dried composite material is then subjected to crosslinking step (c) . This step is needed to crosslink the naturally derived polymer porous sponge constituting the composite material by means of a gaseous crosslinking agent to strengthen the naturally derived polymer porous sponge and to enhance their binding with the biodegradable synthetic polymer mesh or porous sponge, thereby providing elasticity and strength enough to stabilize the porous structure of a desired, crosslinked composite material.

As a crosslinking procedure, there are generally known physical crosslinking methods such as thermal crosslinking and photochemical crosslinking by ultraviolet irradiation, and chemical crosslinking methods by a liquid or gaseous crosslinking agent. Most preferably, a gaseous crosslinking agent is used in this invention.

In the thermal crosslinking or photochemical crosslinking by ultraviolet irradiation, degrees of crosslinking may be limited, and further decomposition or degradation of the biodegradable synthetic polymer constituting the composite material may be caused. In the chemical crosslinking using a liquid crosslinking agent, the naturally derived polymer may be dissolved during the crosslinking. Additionally, to prevent the naturally derived polymer from dissolving into the solution of crosslinking agent, the photochemical or thermal crosslinking may be applied before crosslinking using such a liquid crosslinking agent. In this case, however, light or heat may lead to the decomposition or degradation of the biodegradable synthetic polymer as described above.

Accordingly, in the present invention, the procedure using a gaseous crosslinking agent is preferred since the naturally derived polymer can be crosslinked in a desired manner to form a 3D (three-dimentional)-structure without degradation or decomposition of the biodegradable synthetic polymer, while enhancing the binding with the biodegradable synthetic polymer mesh or porous sponge. Thus, a cross-linked composite material can be obtained which has the desired strength and elasticity.

The crosslinking agent used according to the present invention may be any agent which is conventionally known; preferably, aldehydes including glutaraldehyde, formaldehyde, and paraformaldehyde, especially glutaraldehyde, may be used herein.

In the crosslinking step according to the invention, the crosslinking agent is used in the form of a gas as described above. Specifically, crosslinking of the naturally derived polymer porous sponge is conducted for a given time at a given temperature under an atmosphere saturated with an aqueous crosslinking agent solution at a given concentration.

The crosslinking temperature may be set to such a range that the biodegradable synthetic polymer mesh or porous sponge will not melt and the crosslinking agent can vaporize, and usually set to 20 - 50 °C.

The crosslinking time, although depending upon types of crosslinking agent used and crosslinking temperatures, is preferably set to such a range that the hydrophilicity and biodegradability of the naturally derived polymer porous sponge are not adversely affected and the crosslinking may be conducted to such an extent that the composite material may not dissolve when used for cell culture and implantation.

The shorter the crosslinking time, the poorer the crosslinking immobilization, so that the naturally derived polymer porous sponge may be dissolved during cell seeding. With longer crosslinking times, higher degrees of the crosslinking may be achieved. However, if too long, the hydrophilicity may be decreased and the biodegradability may become slower. Thus, the crosslinking time should be adjusted so as to achieve efficient fixation while do not reduce the hydrophilicity and biodegradability so remarkably, and may be about 2-8 hours, and preferably 3-5 hours when using glutaraldehyde vapor saturated with 25% glutaraldehyde aqueous solution at 37 °C.

In the method for preparing the composite material according to the invention, the biodegradable synthetic polymer porous sponge may be first formed into any 3D shape, for example, a cylindrical form, corresponding to the region to be implanted, and then the naturally derived polymer porous sponge may be formed in the pores of the porous sponge. Such method is simple in operation and good in mechanical strength, while chondrocytes or their progenitor cells differentiating thereto may sometimes be difficult to be delivered into the inmost of the pores of the naturally derived polymer porous sponge, so causing to decrease the cell seeding density of these cells.

The form of the composite material according to the invention may include, but not limited to, a sheet form, a laminate form, and a roll form. The preferred form of the composite material is a sheet according to the invention. On the surface and/or in the openings of such a sheet form of the biodegradable synthetic polymer mesh or porous sponge, that is, within mesh or pores thereof, the naturally derived polymer porous sponge is to be formed. The total thickness of the sheet form composite material may be 0.1 - 5 mm, preferably 0.1 - 1 mm. The thickness of the naturally derived polymer porous sponge can appropriately be adjusted, but preferably be substantially identical to that of the biodegradable synthetic polymer mesh or porous sponge. The porosity of such porous sponges of naturally derived polymer is usually 90 % or more, preferably 95 % to 99.9 %. As used herein, the term "sheet" may encompass a film and membrane form.

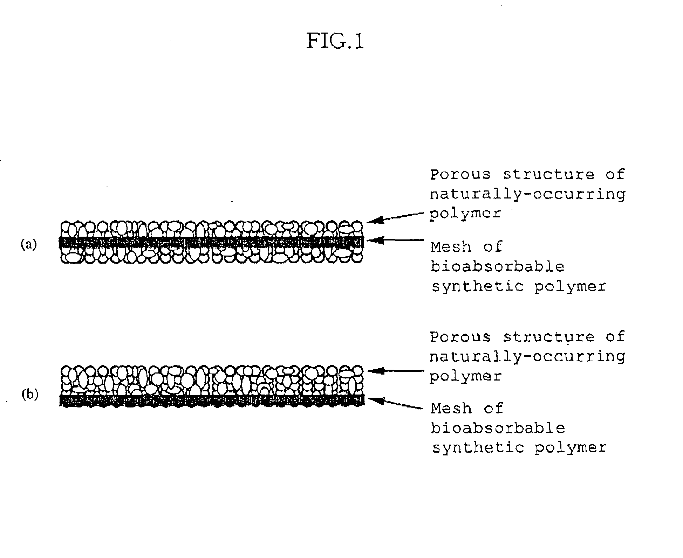

In a preferred embodiment, to produce the sheet form composite material, as illustrated in Fig. 1(a), according to the invention, a sheet form biodegradable synthetic polymer mesh or porous sponge is placed in the center of the aqueous solution of the naturally derived polymer, and frozen, and then freeze-dried. This allows a sheet form composite material to form with a biodegradable synthetic polymer mesh or porous sponge sandwiched in a naturally derived polymer porous sponge. In another preferred embodiment, when a sheet form biodegradable synthetic polymer mesh or porous sponge is placed to be frozen on the top or bottom surface of the aqueous naturally derived polymer solution, a sheet form composite material as illustrated in Fig. 1(b) is formed in which one side is the biodegradable synthetic polymer mesh or porous sponge and the other side is the naturally derived polymer porous sponge.

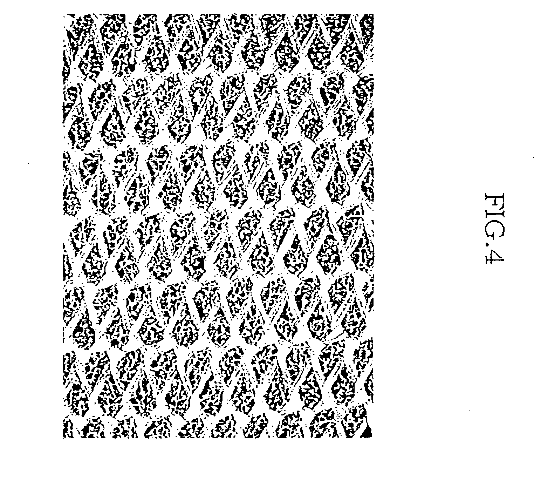

Figs. 1(a) and 1(b) schematically illustrate composite materials such that the naturally derived polymer porous sponge is formed on the surface of the biodegradable synthetic polymer mesh or porous sponge. However, it should be noted that actually the former is also formed within the mesh or pores of the latter as clearly seen from the electron micrograph of Fig. 4.

The chondrocytes or their progenitor cells differentiating thereto used in the invention may be isolated from organism tissues using a conventional method.

For example, the chondrocytes are treated with enzymes (including collagenase, trypsin, lipase and proteinase) to decompose extracellular matrices, mixed with serum medium, and centrifuged to isolate the cells. The isolated chondrocytes are seeded into a culture flask and incubated in DMEM medium (DMEM serum medium) containing 10% fetal calf serum, 4500 mg/L glucose, 584 mg/L glutamin, 0.4 mM proline, and 50 mg/L ascorbic acid. Until an adequate number of cells is obtained, these cells are subcultured through 2 or 3 passages, and the resulting subcultured cells are recovered by trypsinization to obtain a cell suspension for seeding.

The progenitor cells differentiating into chondrocytes are isolated by centrifuging a bone marrow extract directly or by using a density gradient centrifugation with a percoll density gradient medium. These cells are seeded into an culture flask, and subcultured through 2 or 3 passages in DMEM serum medium to an adequate number of cells. The subcultured cells are recovered by trypsinization to obtain a cell suspension for seeding.

To seed chondrocytes or their progenitor cells differentiating thereto in the composite material according to the present invention, the composite material is first wetted with a small amount of culture medium and then impregnated with the cell suspension for seeding. Alternatively, the composite material may be directly impregnated with such a cell suspension for seeding.

The cell density of the cell suspension for seeding is preferably 1 x 10⁶ - 5 x 10⁷ cells/ml, and the volume of cell suspension seeded is preferably more than the volume of the composite material.

The implant for regenerating cartilage tissues according to this invention may be obtained by impregnating the composite material with the cell suspension for seeding, adding a culture medium, and culturing and proliferating the chondrocytes in the composite material in DMEM serum medium at 37 °C under an atmosphere of 5% CO₂ in an incubator.

For progenitor cells, an additional step is needed for differentiating them into chondrocytes. The implant of the invention may be obtained by impregnating the composite material with a cell suspension for seeding containing the progenitor cells differentiating to the chondrocytes, culturing and proliferating them in a culture medium such as DMEM serum medium for 1 to 2 weeks, and further incubating them in a differentiation condition such as DMEM medium containing 4500 mg/L glucose, 584 mg/L glutamin, 0.4 mM proline, and 50 mg/L ascorbic acid as well as dexamethasone and transforming growth factor - β3 (TGF-β3) for 1 to 2 weeks to differentiate them.

One example of the methods for obtaining an implant for regenerating cartilage tissues by seeding chondrocytes or their progenitor cells differentiating thereto onto such a sheet form of composite material in the preferred embodiment of the invention will hereinbelow be specifically described.

The sheet form composite material according to the present invention is placed into a clean sterilized vessel, for example, a dish, and wetted with a small amount of culture medium, followed by adding dropwise the cell suspension for seeding.

The seeding may be repeated twice or more, with once or twice being preferred. When the cells are seeded twice, it should be done on one side of the sheet for the first time, and before doing the second time the sheet form composite material is turned out. The interval between the first and second seeding is preferably 24 hours.

During the seeding step, in order for the seeded cells not to be leaked out of the sheet form composite material, the edges of the sheet is preferably surrounded with a ring, such as rubber ring.

Then, the sheet form composite material in which the cell suspension has been impregnated is incubated in an incubator for additional 4 hours at 37 °C under an atmosphere of 5% CO₂. Afterward, the rubber ring is removed, and a large amount of the culture medium is added, followed by further incubation, resulting in an implant for cartilage tissue regeneration.

The advantage of using the sheet form composite material in the present invention, is ascribable to the fact that a thinner porous sponge of naturally derived polymer such as collagen sponge formed within the composite material. In such a thinner porous sponge, the seeded cell suspension can be impregnated within pores of the porous sponge without leakage, resulting in a higher density and an evener distribution of the cells held within the composite material and more rapid and efficient cartilage tissue regeneration.

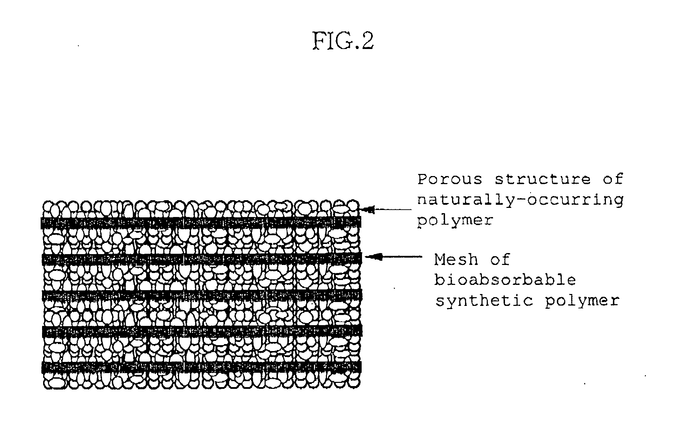

When the composite material seeded with chondrocytes or their progenitor cells differentiating thereto is used in the sheet form, it is possible for a thinner cartilage tissue to be regenerated. Also, it is possible for the composite material seeded with such cells to be used in a laminate form as shown in Fig. 2. The thickness of the cartilage regenerated in this case can be adjusted by the number of laminated sheets of the composite material. In Fig. 2, as the cells are seeded within each sheet of the laminate form of the composite material, the density and distribution of seeded cells in the overall composite material is as high and even as those in one sheet. Therefore, using such a laminated composite material of the present invention as a supporting scaffold of the implant for tissue regeneration, in vivo implantation thereof will allow the cartilage tissue to be well regenerated.

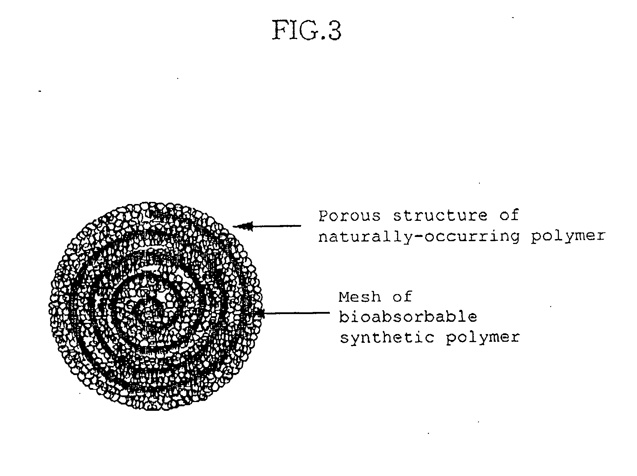

As illustrated in Fig. 3, the sheet form composite material seeded with the cells can also be rolled to take a roll form. In this case, the length of the regenerated cartilage can be adjusted by a roll height, and its diameter can be adjusted by the number of rolling. Alternatively, in the present invention, the sheet form composite material can appropriately be shaped and then assembled so as to conform with the form of the deficient-part of the cartilage tissue to be regenerated.

To obtain various forms of implants comprising a laminate form or roll form composite material, the incubation is preferably continued for 5 days through 2 weeks before molding it into such forms.

### EXAMPLES

The present invention is further illustrated by the following non-limiting examples:

### Example 1

A mesh of poly(D,L-lactic-co-glycolic acid) (PLGA; obtained from Ethico, INC. under Vicryl Knitted Mesh, known as a biodegradable polymer with high mechanical strength, was dipped into 0.5wt% aqueous acidic solution (pH3.0) of bovine atelocollagen I, and frozen at -80 °C for 12 hours. The frozen material was then freeze-dried for 24 hours in vacuo (0.2 Torr) to produce an uncross-linked composite material comprising PLGA mesh and collagen sponge in the form of a sheet.

The resultant uncross-linked composite material was treated with the glutaraldehyde vapor saturated with 25wt% aqueous glutaraldehyde solution at 37 °C for 4 hours, and then washed with phosphate buffer 10 times. Additionally, the material was dipped in 0.1 M aqueous glycine solution for 4 hours, washed 10 times with phosphate buffer and thrice with distilled water, and frozen at -80 °C for 12 hours. It was freeze-dried in vacuo (0.2 Torr) for 24 hours to obtain a crosslinked composite material comprising collagen sponge and PLGA mesh in the form of a sheet.

This material was coated with gold, and their structure was observed under a scanning electron microscope (SEM). The result is shown in Fig. 4. As shown in Fig. 4, collagen sponge is formed within the interstices of PLGA mesh.

### Example 2

The sheet form crosslinked composite material of the PLGA mesh and collagen sponge obtained in Example 1 was cut into a sample of 5.0 mm width x 20.0 mm length. This sample was then dipped in 2-[4-(2-hydroxyethyl)-1-piperazinyl]ethanesulfonic acid (HEPES) buffer (pH7.4). The wet sample was subjected to static tensile test. The results are shown in Table 1.

**Table 1**

| Test sample | Static Young's modulus(MPa) |
|---|---|
| PLGA-collagen | 35.42±1.43 |
| PLGA* | 35.15±1.00 |
| Collagen sponge** | 0.02±0.00 |

| | |
|---|---|
| * Comparison sample: PLGA mesh used in Example 1 | |
| ** Comparison sample: Cross-linked collagen sponge obtained by treating the 0.5wt% aqueous acidic solution (pH3.0) of bovine atelocollagen I as in Example 1 | |

As seen from Table 1, the crosslinked composite material of collagen sponge and PLGA mesh according to the invention exhibits a significantly higher tensile strength than the naturally derived material consisting of collagen sponge as dipped with HEPES buffer solution, and a similar tensile strength as with the PLGA mesh.

### Example 3

The sheet form crosslinked composite material of collagen sponge and PLGA mesh prepared in Example 1 was sterilized by ethylene oxide gas.

On the other hand, a biopsy was shaven off from the bovine elbow-joint cartilage by a scalpel, cut into fine pieces, and incubated in a DMEM medium containing 0.2 (w/v) % collagenase at 37 °C for 12 hours. Further, the supernatant obtained by filtration with a nylon filter of 70 µm in pore size was centrifuged at 2000 rpm for 5 minutes, and then washed twice with a DMEM serum medium containing 10% fetal bovine serum and some antibiotics to provide chondrocytes of the bovine elbow-joint cartilage. The resultant chondrocytes were cultured in DMEM serum medium at 37 °C under an atmosphere of 5% CO₂. The chondrocytes after 2 cycles of subculture were separated and collected with 0.025% trypsin/0.01% EDTA/PBS (-) to prepare a cell suspension at 1 x 10⁷ cells/ml.

Next, the sheet form crosslinked composite material of collagen sponge and PLGA mesh was sterilized by ethylene oxide gas and wetted with DMEM serum medium. The edges of the composite material (membrane) were surrounded with a rubber ring and 1.3 ml/cm² of the cell suspension was dropwise added. The material was static-cultured in an incubator at 37 °C under a 5% CO₂ atmosphere for 4 hours. Then, the rubber ring was taken off, and a large amount of culture medium was added followed by further incubation. The medium was exchanged every 3 days.

After one-week incubation, the composite material was implanted subcutaneously into the nude-mouse dorsa. Six (6) weeks after implantation, a specimen was sampled, and stained with HE (haematoxylin and eosin) or safranin-O. The m-RNA was extracted from the specimen and analysed for type II collagen and aggrecan expression, characteristic of the articular cartilage tissue, using RT-PCR.

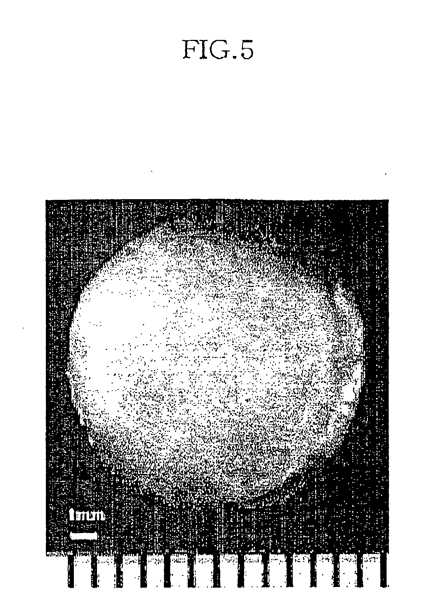

As seen from Fig. 5, the specimen from the subcutaneous implant into the mouse dorsa had a gloss surface after 6 weeks and showed opaque white color.

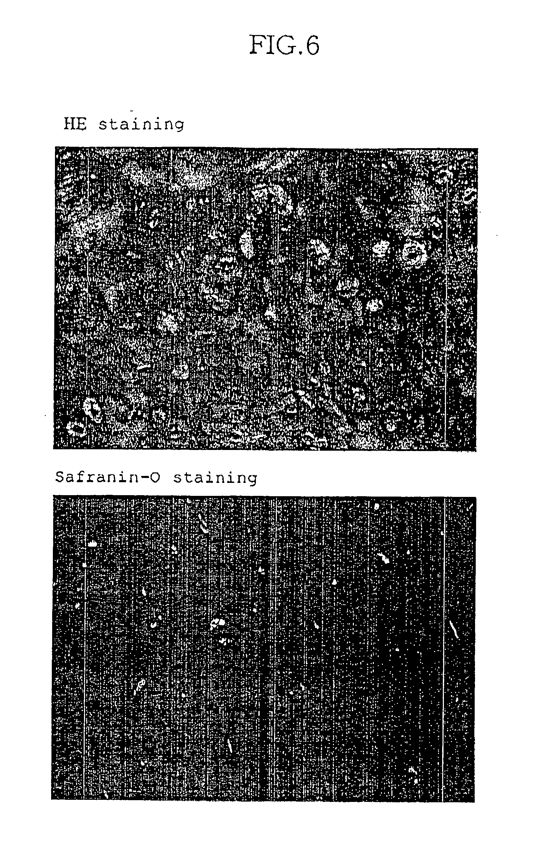

Further, as shown in Fig. 6, rounded cells in lacunae and Safranin-O stained-extracellular matrices were observed in the specimen stained with HE or safranin-O. Still further, among the extracted m-RNA samples from the specimen, the articular cartilage-specific genes, such as type II collagen and aggrecan, were detected, indicating that the regenerated tissue was an articular cartilage tissue.

While the invention has been described in detail with reference to certain preferred embodiments, it is appreciated that many variations and modifications may be made by those skilled in the art within the scope of the present invention as defined in the appended claims. For example, although the inventive implant has been applied to the regeneration of cartilages (using chondrocytes) in the preferred embodiments, it should be understood that the invention may be also applied to produce implants of other tissues such as bone, blood vessels, ligament, skin, bladder, heart valve, and others, by combining the composite materials according to the invention and their respective cells or progenitor cells such as osteoblasts, muscle cells, epithelial cells, fibroblasts, and mesenchymal stem cells by the same approach of the present invention.

## Claims

1. A composite material comprising a mesh or porous sponge of a biodegradable synthetic polymer and a porous sponge of a naturally derived polymer formed on and/or in the mesh or porous sponge, wherein the composite material is cross-linked.

2. The composite material according to claim 1, which is in the form of a sheet.

3. The composite material according to claim 2, which is in the form of a laminate or roll.

4. The composite material according to claim 1, comprising a poly(D,L-lactic-co-glycolic acid) mesh and a collagen sponge, which is cross-linked.

5. A scaffold for supporting a cell, comprising the material according to any one of claims 1 to 4.

6. The scaffold according to claim 5, for supporting chondrocytes or progenitor cells differentiating thereto.

7. An implant for use in cartilage tissue regeneration, comprising chondrocytes or progenitor cells differentiating thereto and a scaffold which comprises the material according to any one of claims 1 to 4.

8. A method for preparing a composite material according to any one of claims 1 to 4, comprising
(a) depositing and impregnating a solution of a naturally derived polymer in a mesh or porous sponge of a biodegradable synthetic polymer;
(b) freeze-drying the solution-impregnated mesh or porous sponge; and
(c) treating the resulting composite material with a gaseous chemical crosslinking agent.

## Patentansprüche

1. Eine Materialzusammensetzung, aufweisend ein Netz oder einen porösen Schwamm aus einem biologisch abbaubaren synthetischen Polymer und einen porösen Schwamm eines auf natürlichem Weg erhaltenen Polymers, ausgebildet auf und/oder in dem Netz oder porösen Schwamm, wobei die Materialzusammensetzung vernetzt ist.

2. Die Materialzusammensetzung nach Anspruch 1, welche die Form einer dünnen Platte hat.

3. Die Materialzusammensetzung nach Anspruch 2, welche die Form eines Laminats oder einer Rolle hat.

4. Die Materialzusammensetzung nach Anspruch 1, aufweisend ein Poly(D,L-Milch-co-Glykolsäure)-Netz und einen Kollagen-Schwamm, der vernetzt ist.

5. Ein Gerüst zum Tragen einer Zelle, aufweisend das Material nach einem der Ansprüche 1 bis 4.

6. Das Gerüst nach Anspruch 5 zum Tragen von Knorpelzellen oder sich zu diesen differenzierenden Stammzellen.

7. Ein Implantat zur Verwendung bei der Regeneration von Knorpelgewebe, mit Knorpelzellen oder sich zu diesen differenzierenden Stammzellen und ein Gerüst, welches das Material nach einem der Ansprüche 1 bis 4 aufweist.

8. Ein Verfahren zum Bereitstellen einer Materialzusammensetzung nach einem der Ansprüche 1 bis 4, aufweisend:
(a) Abscheiden und Imprägnieren einer Lösung aus einem auf natürlichem Weg erhaltenen Polymers in einem Netz oder porösen Schwamm aus einem biologisch abbaubaren synthetischen Polymer;
(b) Gefriertrocknen des mit der Lösung imprägnierten Netzes oder porösen Schwamms; und
(c) Behandeln der sich ergebenden Materialzusammensetzung mit einem gasförmigen chemischen Vernetzungsmittel.

## Revendications

1. Matériau composite comprenant un filet ou une éponge poreuse en polymère synthétique biodégradable et une éponge poreuse en un polymère naturellement dérivé formé sur et/ou dans le filet ou l'éponge poreuse, le matériau composite étant réticulé.

2. Matériau composite selon la revendication 1 qui est sous la forme d'une feuille.

3. Matériau composition selon la revendication 2 qui est sous la forme d'un laminé ou d'un rouleau.

4. Matériau composite selon la revendication 1 comprenant un filet en acide poly(D,L-lactique-co-glycolique) et une éponge en collagène, qui est réticulé.

5. Support pour soutenir une cellule, comprenant le matériau selon l'une quelconque des revendications 1 à 4.

6. Support selon la revendication 5, pour soutenir des chondrocytes ou des cellules souches se différenciant en chondrocytes.

7. Implant à utiliser dans la régénérescence du tissu cartilagineux, comprenant des chondrocytes ou des cellules souches se différenciant en chondrocytes et un support qui comprend le matériau selon l'une quelconque des revendications 1 à 4.

8. Procédé pour préparer un matériau composite selon l'une quelconque des revendications 1 à 4, comprenant les étapes consistant à :
(a) déposer et provoquer l'imprégnation d'une solution d'un polymère dérivé naturellement dans un filet ou une éponge poreuse en polymère synthétique biodégradable;
(b) lyophiliser le filet ou l'éponge poreuse imprégnés de solution ; et
(c) traiter le matériau composite ainsi obtenu avec un agent réticulant chimique gazeux.
